# EUROPÄISCHE PATENTSCHRIFT

(11) **EP 0 495 469 B1**
(45) Veröffentlichungstag und Bekanntmachung des Hinweises auf die Patenterteilung: **20.11.1996**
(21) Anmeldenummer: 92100540.1
(22) Anmeldetag: 15.01.1992
(51) Int. Cl.: A61B 3/12

(54) **Ophthalmoskop**
Ophthalmoscope
Ophthalmoscope

(30) Priorität: 18.01.1991 DE 4101356
(43) Veröffentlichungstag der Anmeldung: 22.07.1992
(73) Patentinhaber: Firma Carl Zeiss, D-73446 Oberkochen (DE); CARL ZEISS-STIFTUNG HANDELND ALS CARL ZEISS, D-89518 Heidenheim (Brenz) (DE)
(72) Erfinder: Poxleitner, Martin, W-7923 Königsbronn (DE); Gaida, Gerhard, Dr., W-7080 Aalen (DE)

(56) Entgegenhaltungen:
- EP-A- 0 145 563
- EP-A- 0 223 356
- DE-U- 8 912 757

## Beschreibung

Die vorliegende Erfindung betrifft ein Ophthalmoskop mit einer Abtasteinheit und einem sphärischen Konkavspiegel, der die Abtasteinheit auf das Auge eines Patienten abbildet und in mindestens zwei unterschiedliche Stellungen schaltbar ist.

Ein solches Ophthalmoskop ist aus der EP-OS 0 223 356 bekannt. Es dient zur Abtastung des Augenhintergrundes mit hoher Auflösung. Dazu wird das Licht eines Lasers in zwei zueinander senkrechten Richtungen abgelenkt und auf das Patientenauge abgebildet. Das am Augenhintergrund reflektierte Licht durchleuchtet dieselbe Abtasteinheit in entgegengesetzter Richtung und wird anschließend in einen Beobachtungsstrahlengang gelenkt.

Die Abtasteinheit besteht aus zwei Elementen, die den einfallenden Lichtstrahl jeweils in zwei zueinander senkrechten Richtungen abtasten. Zwischen den beiden Elementen ist eine Abbildungsoptik zur Kompensation des Astigmatismus des gekippten Konkavspiegels vorgesehen.

Zur Änderung des Abtastbereiches und damit zur Änderung des Sehfeldes ist zwischen dem Konkavspiegel und dem Patientenauge ein Teleskop mit einer von 1x verschiedenen Vergrößerung vorgesehen. Durch vertauschen der Teleskoplinsen ist es dann möglich, die Vergrößerung auf den reziproken Wert umzuschalten.

Ein solches Teleskop verursacht jedoch einerseits zusätzliche Reflexe an den Teleskoplinsen, die den Kontrast des beobachteten Bildes herabsetzen. Andererseits verteuern die zusätzlichen Komponenten das gesamte Ophthalmoskop, und das Teleskop vergrößert außerdem dasjenige Geräteteil des Ophthalmoskopes, das auf das Patientenauge auszurichten ist.

Als alternative Möglichkeit zur Änderung des Sehfeldes wird in der EP-OS 0 223 356 bereits vorgeschlagen, den Konkavspiegel in eine zweite Stellung zu bringen, so daß die Abtasteinheit mit einem anderen Abbildungsmaßstab auf das Patientenauge abgebildet wird. Es werden jedoch keine Angaben gemacht, welche Eigenschaften diese Schaltstellungen haben sollen, oder wie die Umschaltung zwischen den Schaltstellungen erfolgen soll. Statt dessen wird gesagt, daß diese Alternative unbequem zu realisieren ist.

Es ist die Aufgabe der vorliegenden Erfindung, ein Ophthalmoskop der eingangs genannten Art anzugeben, bei dem auf einfache und preiswerte Weise der abgetastete Bereich und damit das Sehfeld variiert werden kann, und bei dem gleichzeitig eine möglichst kompakte Bauweise des auf das Patientenauge auszurichtenden Geräteteils möglich ist.

Diese Aufgabe wird erfindungsgemäß durch ein Ophthalmoskop mit den Merkmalen des Anspruches 1 gelöst.

Es wird also gerade die in der EP-OS 0 223 356 als umständlich bezeichnete Möglichkeit zur Umschaltung des Sehfeldes als besonders vorteilhaft erkannt, wenn sie erfindungsgemäß ausgeführt wird. Die Grundlage dafür bildet die Erkenntnis, daß es möglich ist, den Konkavspiegel in derart versetzte Stellungen zu bringen, daß er die Abtasteinheit mit unterschiedlichen Abbildungsmaßstäben abbildet und daß der Astigmatismus des Konkavspiegels in beiden Schaltstellungen dadurch den selben Wert haben kann, daß der Konkavspiegel senkrecht zu seiner Hauptachse verschoben ist. Eine zusätzliche Kippung des Konkavspiegels zwischen beiden Schaltstellungen ist nicht erforderlich. Dadurch kann der Konkavspiegel auf besonders einfache Weise durch eine eindimensionale, lineare Verschiebung in die unterschiedlichen Schaltstellungen gebracht werden. Für diese lineare Verschiebung können entsprechende Führungselemente vorgesehen sein.

Für die Verschiebung kann ein Riemenantrieb vorgesehen sein, der durch einen Elektromotor angetrieben wird und den Konkavspiegel mitführt.

Die erfindungsgemäße Sehfeldumschaltung ist besonders dann vorteilhaft einsetzbar, wenn die Abtasteinheit aus zwei Elementen besteht, die in Lichtwegrichtung hintereinander angeordnet sind und jeweils Abtastbewegungen in zueinander senkrechten Richtungen ausführen. Diese Elemente sind im tangentialen und sagittalen Fokus des Konkavspiegels angeordnet. Der bei einer Änderung des Abbildungsmaßstabes sich ebenfalls ändernde Astigmatismus des Konkavspiegels wird durch die Verschiebung des Konkavspiegels senkrecht zur Hauptachse kompensiert. Eine Änderung des Abstandes zwischen beiden Abtastelementen ist nicht erforderlich, so daß sie fest in einem Geräteteil angeordnet sein können.

Ein Element der Abtasteinheit ist vorzugsweise ein Polygonspiegelscanner, dessen Abtastfrequenz der Zeilenfrequenz eines Monitors entspricht. Der Monitor dient zur Darstellung eines punktweise zusammengesetzten Bildes des Augenhintergrundes.

Prinzipiell sind durch Verschieben des Konkavspiegels beliebige Zwischenwerte des Abbildungsmaßstabes zwischen einem kleinsten und einem größten Wert möglich, wobei diese Grenzwerte durch die mechanischen Begrenzungen des Verschiebeweges gegeben sind. Besonders einfach und vorteilhaft ist es jedoch, wenn lediglich zwei unterschiedliche Abbildungsmaßstäbe einstellbar sind. Dann läßt sich durch einen einfachen, in den Strahlengang ein- und ausschaltbaren Schaltspiegel erreichen, daß die Abtasteinheit in beiden Schaltstellungen auf den selben Bildpunkt abgebildet wird.

Weitere vorteilhafte Ausführungsformen der Erfindung ergeben sich aus den Unteransprüchen 6 - 8.

So ist nach Anspruch 6 das Ophthalmoskop vorzugsweise als konfokales Ophthalmoskop ausgebildet. Es ist dann eine Punktlichtquelle vorgesehen, mit deren Licht der Augenhintergrund abgetastet wird. Beobachtungsseitig ist in einer zur Punktlichtquelle konjugierten Ebene eine Lochblende vorgesehen. Die Abtasteinheit ist in einem gemeinsamen Bereich des Beleuchtungs- und Beobachtungsstrahlenganges angeordnet.

Vorzugsweise ist im gemeinsamen Bereich des Beleuchtungs- und Beobachtungsstrahlenganges eine Einrichtung zur Kompensation der Fehlsichtigkeit des Patientenauges vorgesehen. Über eine Steuerung kann mit Hilfe dieser Kompensationseinrichtung dafür gesorgt werden, daß unabhängig von dem gewählten Abbildungsmaßstab die Lichtstrahlen mit derselben Konvergenz auf die Augenpupille fallen.

Besonders vorteilhaft ist ein Ophthalmoskop mit den zusätzlichen Merkmalen des Anspruchs 8, denn bei dieser Ausführungsform ist dasjenige Geräteteil, das auf das Patientenauge auszurichten ist, besonders kompakt. Das Ophthalmoskop ist dadurch bequem zu bedienen.

Im Folgenden wird ein Ausführungsbeispiel der Erfindung an Hand der Zeichnungen näher erläutert. Im einzelnen zeigen:
- Fig. 1: eine schematische Darstellung des erfindungsgemäßen Ophthalmoskops;
- Fig. 2a und 2b: jeweils eine perspektivische Darstellung des dem Patienten zugewandten Teils des Ophthalmoskopes nach Figur 1 in unterschiedlichen Schaltstellungen des Konkavspiegels;
- Fig. 3: eine schematische Darstellung des Verschiebemechanismus.

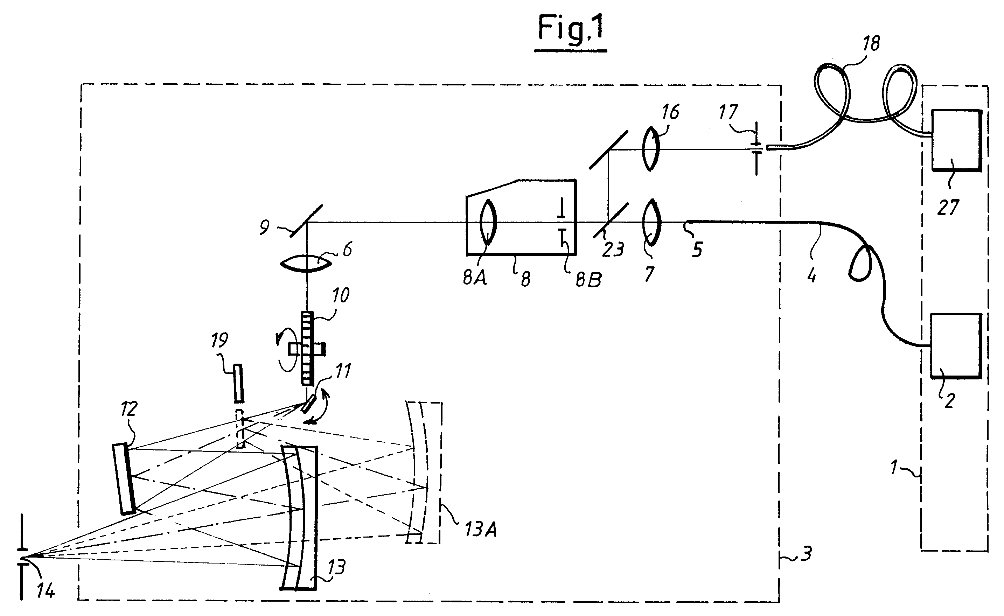

Das Ophthalmoskop nach der Figur 1 besteht aus einem Versorgungsteil (1) und einem relativ zu dem Versorgungsteil (1) beweglichen Geräteoberteil (3), das auf das Patientenauge auszurichten ist. Das Licht eines in dem Versorgungsblock (1) angeordneten Lasers (2) wird über einen Lichtwellenleiter (4) in das Oberteil (3) geführt. Die Stirnfläche (5) des Lichtwellenleiters (4) stellt eine Punktlichtquelle dar. Im weiteren Strahlverlauf ist ein Objektiv (7) vorgesehen, das das Licht der Punktlichtquelle aufsammelt. Dazu ist die Stirnfläche (5) des Lichtwellenleiters (4) im Brennpunkt des Objektivs (7) angeordnet. Hinter dem Objektiv (7) ist eine Einrichtung (8) zur Kompensation der Fehlsichtigkeit des Patientenauges angeordnet. Diese Einrichtung besteht im wesentlichen aus einer Linse (8A) und einer Blende (8B), und ist bereits aus der EP-OS 0 145 563 bekannt. Auf diese Einrichtung (8) braucht daher an dieser Stelle nicht näher eingegangen zu werden.

Hinter der Kompensationseinrichtung (8) ist ein Umlenkspiegel (9) angeordnet, der den Strahlengang über ein weiteres Objektiv (6) auf eine Abtasteinheit lenkt. Die Abtasteinheit besteht aus einem Polygonspiegelscanner (10), der den Lichtstrahl senkrecht zur Zeichenebene ablenkt, und einem Galvanometerscanner (11), der den Lichtstrahl in der Zeichenebene ablenkt. Ein sphärischer Hohlspiegel (13) bildet die Abtasteinheit über einen vorgeschalteten Umlenkspiegel (12) auf die Pupille (14) des Patientenauges ab. Mit Hilfe der Kompensationseinrichtung (8) kann die Konvergenz der einfallenden Lichtstrahlen derart eingestellt werden, daß die Augenlinse des Patienten die einfallenden Lichtstrahlen auf den Augenhintergrund fokussiert.

Der sphärische Konkavspiegel (13) sammelt das am Augenhintergrund zurückreflektierte Licht wieder auf und spiegelt es über den Umlenkspiegel (12), der Abtasteinheit (10, 11) zur Kompensationseinrichtung (8) zurück. Hinter der Kompensationseinrichtung (8) lenkt ein teildurchlässige Spiegel (23) das zurückreflektierte Licht in den Beobachtungsstrahlengang. In diesem Beobachtungsstrahlengang ist ein drittes Objektiv (16) angeordnet, welches das zurückreflektierte Licht in die Ebene einer Lochblende (17) fokussiert. Die Lochblende (17) ist in einer zur Ebene der Stirnfläche (5) und zur Ebene des Augenhintergrundes konfokalen Ebene angeordnet. Der Durchmesser dieser konfokalen Blende (17) ist gerade so gewählt, daß nur das Licht des von der Augenlinse erzeugten Laserfokus, also daß am Augenhintergrund reflektierte Licht, durch die Lochblende (17) transmittiert wird. Das vor oder hinter dem Laserfokus reflektierte oder gestreute Licht wird dagegen von der Lochblende (17) absorbiert. Durch die Lochblende (17) wird dadurch eine Verbesserung des Kontrastes und der Auflösung erzielt. Hinter der Lochblende (17) ist ein zweiter flexibler Lichtleiter (18) vorgesehen, der das durch die Lochblende (17) transmittierte Licht zu einem im Versorgungsblock (1) angeordneten Photomultiplier (27) leitet.

Zur Umschaltung des Abbildungsmaßstabes, bzw. der Abtastwinkel, unter denen die Lichtstrahlen auf die Pupille (14) fallen, ist für den Konkavspiegel (13) eine zweite, gestrichelt dargestellte Schaltstellung vorgesehen, in der der Konkavspiegel mit (13A) bezeichnet ist. Gleichzeitig mit der Umschaltung des Konkavspiegels in die zweite Stellung wird ein Schaltspiegel (19) zwischen der Abtasteinheit und dem Umlenkspiegel (12) in den Strahlengang eingeschaltet, so daß dieser Schaltspiegel (19) den Abtaststrahl auf den sphärischen Konkavspiegel (13A) lenkt. In der zweiten Schaltstellung ist der optische Weg zwischen der Abtasteinheit und dem sphärischen Hohlspiegel (13A) kürzer als der optische Weg zwischen der Abtasteinheit und dem Konkavspiegel (13) in der ersten Schaltstellung. Der Konkavspiegel (13A) bildet daher in der zweiten Schaltstellung die Abtasteinheit mit einem geringeren Abbildungsmaßstab auf die Pupille (14) ab als in der ersten Schaltstellung. Dabei ist wichtig, daß der Konkavspiegel (13, 13A) in beiden Schaltstellungen die Abtasteinheit auf dieselbe Pupille (14) abbildet. Das Geräteoberteil (3) braucht deshalb nach einer vorgenommenen Sehfeldumschaltung nicht neu auf das Patientenauge ausgerichtet zu werden.

Synchron zur Umschaltung des Konkavspiegels (13, 13A) wird die Linse (8A) in der Kompensationseinrichtung (8) so verschoben, daß die Konvergenz bzw. Divergenz des einfallenden Lichtstrahls an der Pupille (14) unabhängig von der Schaltstellung des Konkavspiegels (13, 13A) ist. Dazu ist der Antrieb für den Konkavspiegel (13, 13A) in nicht näher dargestellter Weise mit dem Antrieb für die Linse (8A) gekoppelt. Der Augenhintergrund des Patienten ist dann stets in einer zur Stirnfläche (5) des Lichtwellenleiters (4) konjugierten Ebene. Ebenso sind die Stirnfläche (5) des Lichtwellenleiters (4) und die Lochblende (17) stets in zueinander konjugierten Ebenen angeordnet.

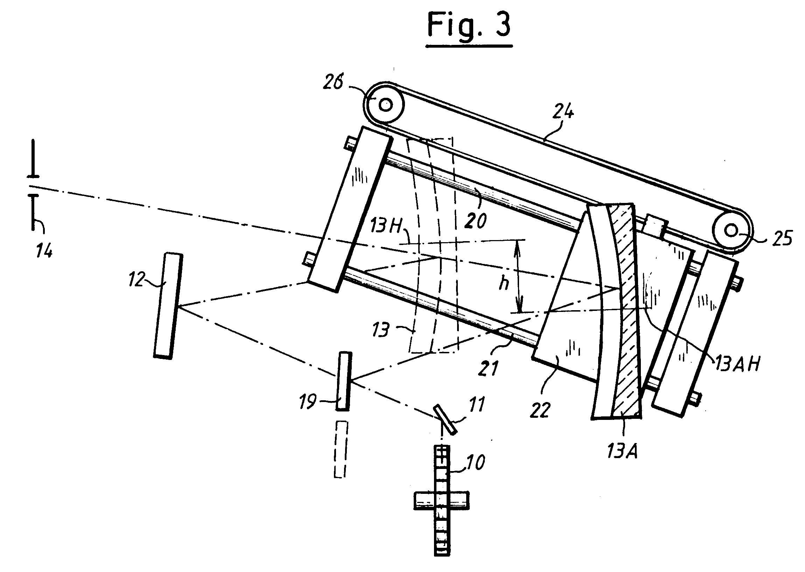

Die Umschaltung des Konkavspiegels (13) erfolgt, wie aus der Figur 3 ersichtlich, durch eine lineare Verschiebung des Spiegels, wobei die Verschiebung sowohl eine Verschiebungskomponente parallel zur Hauptachse (13H, 13AH) als auch eine Verschiebungskomponente senkrecht zur Hauptachse (13H, 13AH) des Konkavspiegels (13, 13A) aufweist. Die einzelnen Bauteile sind in der Figur 3 mit den gleichen Bezugszeichen versehen, wie in der Figur 1. Der Spiegel (13A) ist auf einem Träger (22) montiert, der entlang zweier Führungsschienen (20, 21) motorisch verschiebbar ist. Für die motorische Verschiebung ist ein Riemenantrieb vorgesehen, bei dem ein Zahnriemen (24) über ein auf einer Motorwelle angeordnetes angetriebenes Rad (25) und ein abgetriebenes Rad (26) geführt ist. Der Spiegelträger (22) ist an dem Zahnriemen (24) befestigt und wird mit dem Riemen mitgeführt. Die Verschiebung entlang der Führungsschienen (20, 21) erfolgt dabei so, daß die Hauptachse (13AH) des Konkavspiegels, in der zweiten Schaltstellung um einen Betrag (h) senkrecht zur Hauptachse (13H) in der ersten Schaltstellung verschoben ist. Der Abbildungsmaßstab, mit dem die Abtasteinheit in die Ebene der Pupille (14) abgebildet wird, ist durch die optische Weglänge zwischen der Abtasteinheit und dem sphärischen Hohlspiegel (13, 13A) in der jeweiligen Schaltstellung gegeben. Durch die Verschiebung des Konkavspiegels (13, 13A) wird sein Astigmatismus durch zwei Effekte geändert. Der erste Effekt ist eine Änderung des Astigmatismus, die durch den veränderten Abbildungsmaßstab verursacht ist. Der zweite Effekt ist eine Änderung des Astigmatismus aufgrund unterschiedlicher Winkel, unter denen das Licht in den beiden Schaltstellungen auf den Konkavspiegel (13, 13A) fällt. Die Ursache dafür ist, daß der Spiegel (12) und der Schaltspiegel (19) nicht parallel zueinander ausgerichtet sind. Der Abstand (h), um den der sphärische Spiegel (13, 13A) beim Umschalten senkrecht zu seiner Hauptachse (13H, 13AH) verschoben wird, ist gerade so gewählt, daß in beiden Schaltstellungen der Astigmatismus des Konkavspiegels (13, 13A) gleich ist. Der Polygonspiegelscanner (10) ist dann im sagittalen Fokus und der Galvanometerscanner (11) im tangentialen Fokus des Konkavspiegels (13, 13A) angeordnet. Beide Elemente (10, 11) der Abtasteinheit werden daher in jeder der beiden Schaltstellungen scharf in die Ebene der Pupille (14) abgebildet.

Die Formeln zur Berechnung der Lagen des tangentialen und des sagittalen Fokus eines gekippten Hohlspiegels sind beispielsweise in dem Lehrbuch von Jenkins and White "Fundamentals of Optics", Mc Graw-Hill, 1950, S. 92 beschrieben. Anhand dieser Formeln und den Forderungen, daß die Pupille (14) in beiden Schaltstellungen an derselben Stelle liegt, daß die Elemente (10, 11) der Abtasteinheit in beiden Schaltstellungen an den selben Orten angeordnet sind und daß die Maßstäbe, mit denen der Konkavspiegel (13, 13A) die beiden Elemente (10, 11) der Abtasteinheit auf die Pupille (14) abbildet, fest vorgegeben sind, kann der erforderliche Abstand (h) sowie die Position und die Orientierung des Schaltspiegels (19) eindeutig mit Hilfe geometrischer Überlegungen konstruiert werden.

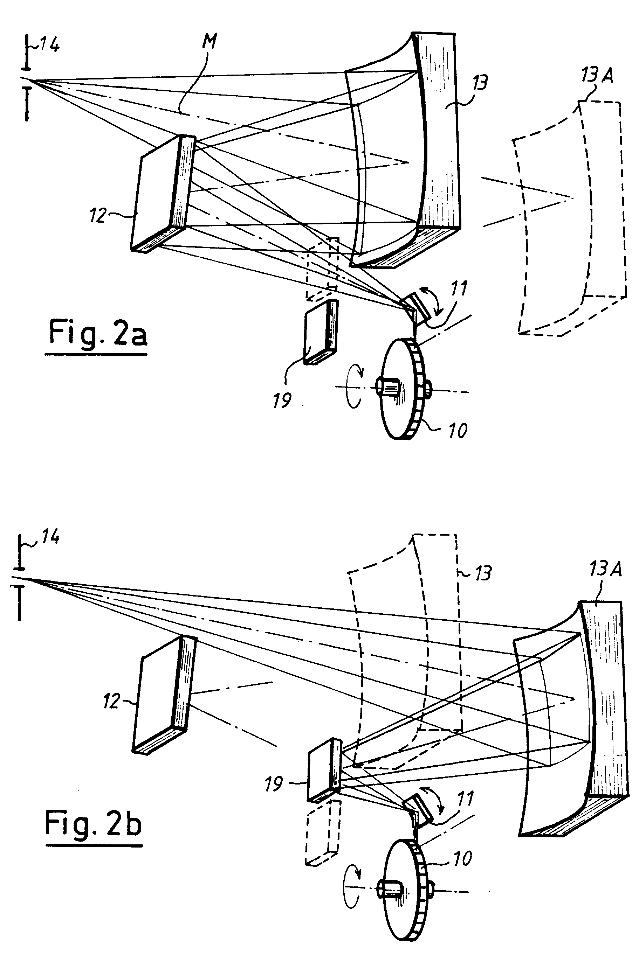

In den Figuren 2a und 2b sind wiederum gleiche Komponenten wie in den Figuren 1 und 3 mit denselben Bezugszeichen versehen. In der Fig. 2a ist der Konkavspiegel (13) in die erste Stellung geschaltet. Der Schaltspiegel (19) ist dabei aus dem Strahlengang herausgeschaltet. In dieser Schaltstellung wird die Abtasteinheit mit einem Vergrößerungsmaßstab von √2 auf die Pupille (14) abgebildet.

In der Figur 2b ist der Konkavspiegel (13A) in die zweite Schaltstellung geschaltet. Der Umlenkspiegel (19) ist jetzt in den Strahlengang eingeschaltet. In dieser Schaltstellung wird die Abtasteinheit mit einem Vergrößerungsmaßstab von 1/ √2 auf die Pupille (14) abgebildet. Wie insbesondere aus der Figur 2a sehr deutlich hervorgeht, schneidet die Achse (M), um die die Lichtstrahlen abgelenkt werden, den sphärischen Spiegel (13, 13A) in seinen beiden Schaltstellungen an verschiedenen Stellen. Durch diese Verschiebung des sphärischen Spiegels senkrecht zu seiner Hauptachse wird in den beiden Schaltstellungen die astigmatische Fokusdifferenz gleich.

## Patentansprüche

1. Ophthalmoskop mit einer Abtasteinheit und einem sphärischen Konkavspiegel, der die Abtasteinheit in eine für das Auge des Patienten vorgesehene Stelle abbildet und in mindestens zwei unterschiedliche Stellungen schaltbar ist, wobei in den unterschiedlichen Schaltstellungen die optischen Weglängen zwischen der Abtasteinheit (10, 11) und dem Konkavspiegel (13, 13A) unterschiedlich sind, dadurch gekennzeichnet, daß die unterschiedlichen Schaltstellungen durch eine lineare Verschiebung des Konkavspiegels (13, 13A) in einander überführbar sind und daß in den unterschiedlichen Schaltstellungen des Konkavspiegels (13, 13A) die Abtasteinheit (10, 11) in die selbe Stelle (14) abgebildet ist, wozu ein zwischen der Abtasteinheit (10, 11) und dem Konkavspiegel (13, 13A) in den Strahlengang einschaltbarer Schaltspiegel (19) vorgesehen ist.

2. Ophthalmoskop nach Anspruch 1, dadurch gekennzeichnet, daß die Abtasteinheit aus zwei Elementen (10, 11) besteht, die in Lichtrichtung hintereinander angeordnet sind und das Patientenauge in zwei zueinander senkrechten Richtungen abtasten, und daß der Abstand beider Elemente (10, 11) fest ist.

3. Ophthalmoskop nach Anspruch 2, dadurch gekennzeichnet, daß der Konkavspiegel (13, 13A) beide Elemente (10, 11) der Abtasteinheit gleichzeitig scharf auf die Stelle (14) abbildet.

4. Ophthalmoskop nach Anspruch 3, dadurch gekennzeichnet, daß ein Element (10) der Abtasteinheit ein Polygonspiegelscanner ist.

5. Ophthalmoskop nach Anspruch 4, dadurch gekennzeichnet, daß Führungselemente (20, 21) für die lineare Verschiebung des Konkavspiegels (13, 13A) vorgesehen sind.

6. Ophthalmoskop nach Anspruch 1, dadurch gekennzeichnet, daß die Abtasteinheit (10, 11) in einem Teil des Beleuchtungs- und Beobachtungsstrahlenganges angeordnet ist, daß die Abtasteinheit (10, 11) den Augenhintergrund mit dem Licht einer Punktlichtquelle (5) abtastet, daß in einer zur Punktlichtquelle (5) konjugierten Ebene eine Lochblende (17) angeordnet ist, und daß hinter der Lochblende (17) ein Detektor (27) zur Detektion des am Augenhintergrund reflektierten Lichts angeordnet ist.

7. Ophthalmoskop nach Anspruch 6, dadurch gekennzeichnet, daß im gemeinsamen Beleuchtungs- und Beobachtungsstrahlengang eine Einrichtung (8) zur Kompensation der Fehlsichtigkeit des Patientenauges vorgesehen ist.

8. Ophthalmoskop nach Anspruch 7, dadurch gekennzeichnet, daß die Punktlichtquelle (5), die Abtasteinheit (10, 11), der Konkavspiegel (13, 13A) und die zur Punktlichtquelle (5) konfokale Lochblende (17) in einem ersten Geräteteil (3) und der in Lichtrichtung hinter der Lochblende (17) angeordnete Detektor (27) in einem zweiten Geräteteil (1) angeordnet sind, und daß der erste Geräteteil (3) relativ zum zweiten Geräteteil (1) beweglich ist, und daß eine flexible Lichtleitfaser (18) vorgesehen ist, die das durch die Lochblende (17) transmittierte Licht zum Detektor (27) leitet.

9. Ophthalmoskop nach Anspruch 8, dadurch gekennzeichnet, daß für die Verschiebung des Konkavspiegels (13, 13A) ein elektromotorischer Antrieb vorgesehen ist.

## Claims

1. Ophthalmoscope with a scanning unit and a spherical concave mirror which images the scanning unit onto a position destined for the eye of a patient and which is switchable into at least two different positions whereby in the at least two different switching positions the optical path lengths between the scanning unit (10, 11) and the concave mirror (13, 13A) are different, characterized in that the concave mirror (13, 13A) can be brought from one switching position to the other by a linear displacement of the concave mirror (13, 13A) and that in the different switching positions of the concave mirror (13, 13A) the scanning unit (10, 11) is imaged onto the same position (14) for which a switchable mirror (19) is provided which is insertable into the beam path between the scanning unit (10, 11) and the concave mirror (13, 13A).

2. Ophthalmoscope of claim 1, characterized in that the scanning unit comprises two elements (10, 11) which are arranged one behind the other in the direction of light propagation and which scan the eye of a patient within two perpendicular directions and whereby both elements (10, 11) are separated by a fixed spacing.

3. Ophthalmoscope of claim 2, characterized in that the concave mirror (13, 13A) images both elements (10, 11) of the scanning unit simultaneously sharply onto the position (14).

4. Ophthalmoscope of claim 3, characterized in that one element (10) of the scanning unit is a polygon-mirror scanner.

5. Ophthalmoscope of claim 4, characterized in that guiding elements (20, 21) are provided for the linear displacement of the concave mirror (13, 13A).

6. Ophthalmoscope of claim 1, characterized in that the scanning unit (10, 11) is arranged within a portion of the illuminating and viewing optical beam path, that the scanning unit scans the ocular fundus by the light of a point light source (5), that a pin-hole diaphragm (17) is arranged within a plane conjugated to the point light source (5) and that behind the pin-hole diaphragm (17) a detector (27) is arranged for detecting the light reflected at the ocular fundus.

7. Ophthalmoscope of claim 6, characterized in that a unit (8) for compensating for the ametropia of the eye of the patient is provided within the common illuminating and viewing beam path.

8. Ophthalmoscope of claim 7, characterized in that the point light source (5), the scanning unit (10, 11), the concave mirror (13, 13A) and the pin-hole diaphragm (17) confocal to the point light source (5) are arranged within a first portion (3) of the device and that the detector (27) arranged, in the direction of light, downstream of the pin-hole diaphragm (17) is arranged within a second portion (1) of the device and that the first portion (3) and the second portion (1) are movable with respect to one another and that a flexible light conducting fiber (18) is provided for guiding the light transmitted through the pin-hole diaphragm (17) to the detector (27).

9. Ophthalmoscope of claim 8, characterized in that an electric motor driven drive is provided for the displacement of the concave mirror (13, 13A).

## Revendications

1. Ophtalmoscope avec une unité de balayage et un miroir concave sphérique qui reproduit l'image de l'unité de balayage en un point prévu pour l'oeil du patient et qui est orientable dans deux positions différentes au moins où le trajet optique entre l'unité de balayage (10, 11) et le miroir concave (13, 13A) diverge en conséquence, caractérisé en ce que ces deux positions de réglage peuvent être interverties par simple déplacement linéaire du miroir concave (13, 13A) et que l'unité de balayage (10, 11) est projetée au même endroit (14) dans les diffèrentes positions du miroir concave (13, 13A) au moyen d'un miroir (19) qui est intercalable à cet effet dans le faisceau lumineux entre l'unité de balayage (10, 11) et le miroir concave (13, 13A).

2. Ophtalmoscope selon la revendication 1, caractérisé en ce que l'unité de balayage comprend deux éléments (10, 11) qui sont disposés l'un derrière l'autre dans le sens de la lumière et qui balaient l'oeil du patient dans deux directions perpendiculaires l'une par rapport à l'autre, et en ce que l'écart entre les deux éléments (10, 11) est fixe.

3. Ophtalmoscope selon la revendication 2, caractérisé en ce que le miroir concave (13, 13A) reproduit simultanément une image nette des deux éléments (10, 11) de l'unité de balayage au point (14).

4. Ophtalmoscope selon la revendication 3, caractérisé en ce qu'un élément de l'unité de palpage (10) est un analyseur à balayage à miroir polygonal.

5. Ophtalmoscope selon la revendication 4, caractérisé en ce que des éléments de guidage (20, 21) sont prévus pour déplacer linéairement le miroir concave (13, 13A).

6. Ophtalmoscope selon la revendication 1, caractérisé en ce que l'unité de balayage (10, 11) est disposée dans une partie du trajet des faisceaux d'éclairage et d'observation, que l'unité de balayage (10, 11) explore le fond de l'oeil à l'aide de la lumière d'une source ponctuelle (5), qu'un diaphragme à trou central (17) est placé dans un plan conjugué avec la source lumineuse ponctuelle (5) et qu'un détecteur (27) se trouve derrière le diaphragme (17) pour capter la lumière réfléchie par le fond de l'oeil.

7. Ophtalmoscope selon la revendication 6, caractérisé en ce qu'un dispositif (8) est prévu pour compenser l'amétropie de l'oeil du patient dans le trajet commun des faisceaux d'éclairage et d'observation.

8. Ophtalmoscope selon la revendication 7, caractérisé en ce que la source lumineuse ponctuelle (5), l'unité de palpage (10, 11), le miroir concave (13, 13A) et le diaphragme à trou central (17) confocal par rapport à la source de lumière ponctuelle (5) sont disposés dans une première partie instrumentale (3), tandis que le détecteur (27) placé derrière le diaphragme (17) dans le sens de la lumière occupe une seconde partie instrumentale (1), et en ce que la première partie instrumentale (3) est mobile par rapport à la seconde partie instrumentale et qu'une fibre optique souple (18) est disponible pour conduire la lumière transmise à travers le diaphragme à trou central (17) jusqu'au détecteur (27).

9. Ophtalmoscope selon la revendication 8, caractérisé en ce qu'un actionneur à commande électrique assure le déplacement du miroir concave (13, 13A).
